# EUROPÄISCHE PATENTANMELDUNG

(11) **EP 0 721 775 A2**
(43) Veröffentlichungstag der Anmeldung: **17.07.1996**
(21) Anmeldenummer: 95118408.4
(22) Anmeldetag: 23.11.1995
(51) Int. Cl.: A61K 7/48, A61K 31/19, A61K 31/215, A61K 31/07

(54) **Gegen unreine Haut, leichte Formen der Akne und grampositive Bakterien wirksame Wirkstoffkombinationen**

(30) Priorität: 13.12.1994 DE 4444237
(71) Anmelder: Beiersdorf Aktiengesellschaft, D-20245 Hamburg (DE)
(72) Erfinder: Schönrock, Uwe, Dr., D-22844 Norderstedt (DE); Wolf, Florian, Dr., D-20251 Hamburg (DE); Traupe, Bernd, D-22457 Hamburg (DE); Christiansen, Michael, D-25436 Tornesch (DE); Steinke, Sigrid, D-21075 Hamburg (DE)

(57) **Zusammenfassung**

Kosmetische und/oder dermatologische Wirkstoffkombinationen, bestehend aus
a) einer wirksamen Menge an Salicylsäure und/oder einer wirksamen Menge an einer oder mehreren α-Hydroxycarbonsäuren und/oder einer wirksamen Menge an einer oder mehreren α-Ketocarbonsäuren
b) einem oder mehreren Stoffen, gewählt aus der Gruppe der Monoglycerinmonocarbonsäure-monoester, der Diglycerin-monocarbonsäure-monoester sowie der Triglycerin-monocarbonsäure-monoester sowie
c) gegebenenfalls einer wirksamen Menge an Retinol und/oder Derivaten des Retinols.

## Beschreibung

Die vorliegende Erfindung betrifft Wirkstoffe und Zubereitungen, solche Wirkstoffe enthaltend, welche gegen unreine Haut wirksam sind.

Bei der unreinen Haut sind neben anderen Einflüssen bakterielle Sekundärinfektionen von ätiologischer Bedeutung. Einer der wichtigsten Mikroorganismen, der in Zusammenhang mit unreiner Haut steht, ist Propionibacterium acnes.

Unreine Haut und/oder Komedonen beeinträchtigen das Wohlbefinden der Betroffenen aber selbst in leichten Fällen. Da praktisch jeder oder jede Jugendliche von unreiner Haut irgendeiner Ausprägung betroffen ist, besteht bei vielen Personen Bedarf, diesem Zustande abzuhelfen.

Aufgabe der vorliegenden Erfindung war es also, einen gegen unreine Haut bzw. Propionibacterium acnes wirksamen Stoff bzw. Stoffkombination zu finden.

Es hat sich überraschenderweise gezeigt, und darin liegt die Lösung der Aufgabe, daß kosmetische und/oder dermatologische Wirkstoffkombinationen, bestehend aus
a) einer wirksamen Menge an Salicylsäure und/oder einer wirksamen Menge an einer oder mehreren α-Hydroxycarbonsäuren der allgemeinen Formel 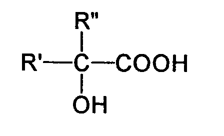 und/oder einer wirksamen Menge an einer oder mehreren α-Ketocarbonsäuren der allgemeinen Formel 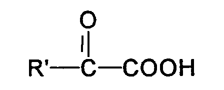 wobei jeweils R' und R'' unabhängig voneinander gewählt werden aus der Gruppe
(a1) H-,
(a2) verzweigtes oder unverzweigtes C₁₋₂₅₋Alkyl-,
(a3) mit einer oder mehreren Carboxylgruppen und/oder Hydroxygruppen und/oder Aldehydgruppen und/oder Oxogruppen (Ketogruppen) substituiertes verzweigtes oder unverzweigtes C₁₋₂₅-Alkyl-
(a4) Phenyl-,
(a5) mit einer oder mehreren Carboxylgruppen und/oder Hydroxygruppen und/oder verzweigten und/oder unverzweigten C₁₋₂₅-Alkylgruppen substituiertes Phenyl-, oder wobei das α-Kohlenstoffatom der α-Hydroxycarbonsäure mit R' und R'' zusammen eine
(a6) unsubstituierte Cycloalkylgruppe mit 3 bis 7 Ringatomen oder eine
(a7) mit einer oder mehreren Carboxylgruppen und/oder Hydroxygruppen und/oder Oxogruppen (Ketogruppen) und/oder verzweigten und/oder unverzweigten C₁₋₂₅-Alkylgruppen substituierte Cycloalkylgruppe mit 3 bis 7 Ringatomen ausbildet und wobei die α-Hydroxycarbonsäure oder die α-Hydroxycarbonsäuren oder die α-Ketocarbonsäure oder die α-Ketocarbonsäuren gegebenenfalls in Form ihrer physiologisch verträglichen Salze und/oder Ethylester und/oder Methylester vorliegen können
und
b) einem oder mehreren Stoffen, gewährt aus der Gruppe der Monoglycerinmonocarbonsäure-monoester, der Diglycerin-monocarbonsäure-monoester sowie der Triglycerin-monocarbonsäure-monoester sowie
c) gegebenenfalls einer wirksamen Menge an Retinol und/oder Derivaten des Retinols
den Mißständen des Standes der Technik abhelfen.

Die Salicylsäure (auch 2-Hydroxybenzoësäure, Spirsäure) ist durch die Struktur 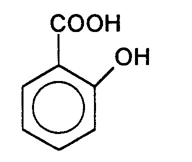
gekennzeichnet. Bekanntermaßen wirkt Salicylsäure antibakteriell und keratolytisch und ist Bestandteil mancher kosmetischen oder pharmazeutischen Zubereitungen.

Die erfindungsgemäßen α-Hydroxycarbonsäuren werden vorteilhaft gewährt aus folgenden Substanzklassen:
(a2) α-Hydroxyfettsäuren, wobei diese wiederum besonders vorteilhaft aus der Gruppe der C₁₀₋₁₈-Alkylcarbonsäuren gewählt werden,
(a3) α-Hydroxyzuckersäuren, aliphatische α-Hydroxyfruchtsäuren,
(a4) unsubstituierte aromatische α-Hydroxycarbonsäuren (z.B. Mandelsäure) bzw.
(a5) substituierte aromatische α-Hydroxycarbonsäuren.

Die unter Punkt (a2) fallenden α-Hydroxyfettsäuren werden besonders vorteilhaft gewährt aus der Gruppe
- α-Hydroxycarbonsäuren, gemäß der Formel 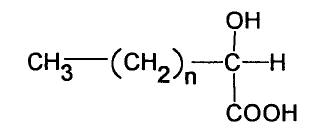 und/oder
- α-Hydroxy-isocarbonsäuren, gemäß der Formel 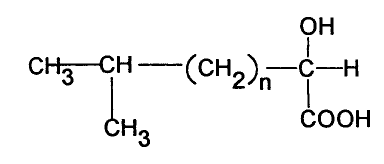 und/oder
- α-Hydroxy-anteisocarbonsäuren, gemäß der Formel 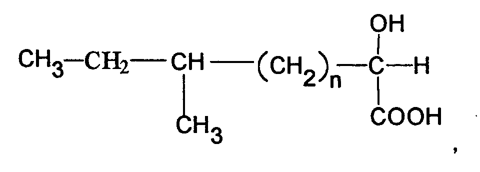 wobei n jeweils eine Zahl von 7 bis 31 darstellt.

Besonders vorteilhaft im Sinne der vorliegenden Erfindung ist, α-Hydroxycarbonsäuren zu verwenden, welche C₁₆-Körper darstellen, die also am α-Kohlenstoffatom eine verzweigte oder unverzweigte C₁₄H₂₉-Kette tragen.

Vorteilhaft ist weiter, Gemische solcher aliphatischen α-Hydroxycarbonsäuren, insbesondere in Form von Wollwachssäuregemischen zu verwenden, in welchen der Gehalt an α-Hydroxycarbonsäuren 20 - 30 Gew.-%, bezogen auf die Gesamtzusammensetzung beträgt.

Die unter Punkt (a3) fallenden α-Hydroxyzuckersäuren werden besonders vorteilhaft gewährt aus der Gruppe der
- Aldonsäuren, z.B. Gluconsäure, Galactonsäure
- Aldarsäuren, z.B. Glucarsäure, Galactarsäure (aber auch die Fruchtsäure Weinsäure, die ebenfalls unter die Definition der Aldarsäure fällt)
- Uronsäuren, z.B. Glucuronsäure, Galacturonsäure
- Glycerinsäure
Die unter Punkt (a3) fallenden aliphatischen α-Hydroxyfruchtsäuren werden besonders vorteilhaft gewählt aus der Gruppe Äpfelsäure, Milchsäure, Citronensäure, Weinsäure.

Äpfelsäure (Hydroxybernsteinsäure) ist durch folgende chemische Struktur gekennzeichnet: 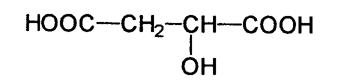
Milchsäure (2-Hydroxypropansäure) ist durch folgende chemische Struktur gekennzeichnet: 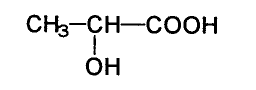
Citronensäure (2-Hydroxy-1,2,3-propantricarbonsäure) ist durch folgende chemische Struktur gekennzeichnet: 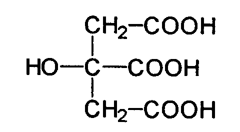
Bekanntermaßen wird Citronensäure zur Pufferung kosmetischer und/oder dermatologischer Zubereitungen, aber auch als Synergist für Antioxidantien in der Haut- und Haarkosmetik verwendet.

Weinsäure (Dihydroxybernsteinsäure) ist durch folgende chemische Struktur gekennzeichnet: 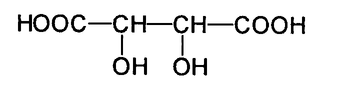
Bevorzugte α-Ketocarbonsäure ist die Brenztraubensäure (α-Oxopropansäure). Sie zeichnet sich durch folgende Struktur aus: 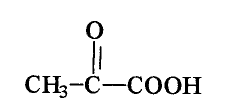
In erstaunlicher Weise hat sich herausgestellt, daß die an sich fakultativen Bestandteile, der oder die Monoglycerin-, Diglycerin- bzw. Triglycerin-monocarbonsäure die synergistische Wirkung des Retinols mit der oder den α-Hydroxysäuren bzw. α-Ketocarbonsäuren noch zusätzlich in ebenfalls synergistischer Weise steigert.

Die erfindungsgemäßen Monoglycerin-monocarbonsäure-monoester werden durch die Strukturen 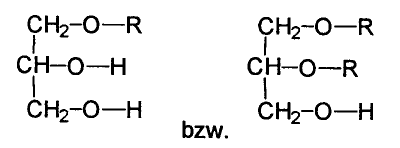
wiedergegeben, wobei R einen verzweigten oder unverzweigten Acylrest mit 6 - 14 Kohlenstoffatomen darstellt. Vorteilhaft wird R gewählt aus der Gruppe der unverzweigten Acylreste. Die diesen Estern Zugrundeliegenden Fettsäuren bzw. Monocarbonsäuren sind die

| | |
|---|---|
| Hexansäure (Capronsäure) | (R = -C(O)-C₅H₁₁), |
| Heptansäure (Önanthsäure) | (R = -C(O)-C₆H₁₃), |
| Octansäure (Caprylsäure) | (R = -C(O)-C₇H₁₅), |
| Nonansäure (Pelargonsäure) | (R = -C(O)-C₈H₁₇), |
| Decansäure (Caprinsäure) | (R = -C(O)-C₉H₁₉), |
| Undecansäure | (R = -C(O)-C₁₀H₂₁), |
| Dodecansäure (Laurinsäure) | (R = -C(O)-C₁₁H₂₃), |
| Tridecansäure | (R = -C(O)-C₁₂H₂₅), |
| Tetradecansäure (Myristinsäure) | (R = -C(O)-C₁₃H₂₇). |

Besonders vorteilhaft stellt R den Octanoylrest (Caprylsäurerest) bzw. den Decanoylrest (Caprinsäurerest) dar, wird also also durch die Formeln

R = -C(O)-C₇H₁₅) bzw. R = -C(O)-C₉H₁₉

repräsentiert.

In dieser Schrift, insbesondere in den Beispielen, wird das Kürze GMCy für Glycerinmonocaprylat und das Kürzel GMC für Glycerinmonocaprinat verwendet.

Bei den in 1-Position des Glycerins veresterten Glycerinestern ist die 2-Position ein Asymmetriezentrum. Erfindungsgemäß aktiv und gleichermaßen von Vorteil sind die 2S- und die 2R-Konfiguration.

Es hat sich als günstig herausgestellt, racemische Gemische der Stereoisomeren zu verwenden.

In den dermatologischen Zubereitungen beträgt der Gehalt an GMCy und/oder GMC vorteilhaft 0,1 - 10,0 Gew.-%, bevorzugt 0,5 bis 7,5 Gew.-%, besonders bevorzugt 1,5 - 5,0 Gew.-%, jeweils bezogen auf das Gesamtgewicht der jeweiligen Formulierung.

Erfindungsgemäß liegen die Di- bzw. Triglycerineinheiten der erfindungsgemäßen Diglycerin-monocarbonsäure-monoester bzw. Triglycerin-monocarbonsäure-monoester als lineare, unverzweigte Moleküle, also über die jeweiligen OH-Gruppen in 1- bzw. 3-Stellung veretherte "Monoglycerinmoleküle" vor.

Ein geringer Anteil zyklischer Di- bzw. Triglycerineinheiten sowie über die OH-Gruppen in 2-Stellung veretherte Glycerinmoleküle kann geduldet werden. Es ist jedoch von Vorteil, solche Verunreinigungen so gering wie nur möglich zu halten.

Die erfindungsgemäßen Monocarbonsäuremonoester sind bevorzugt durch folgende Struktur gekennzeichnet (Substitutionspositionen angegeben): 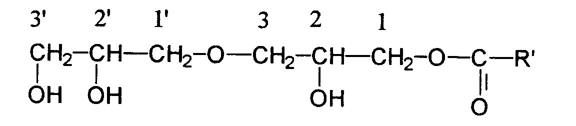
wobei R' einen Kohlenwasserstoffrest, vorteilhaft einen verzweigten oder unverzweigten Alkyl- oder Alkenylrest von 5 bis 17 C-Atomen darstellt.

Die erfindungsgemäßen Monocarbonsäureester des Triglycerins sind bevorzugt durch folgende Struktur gekennzeichnet (Substitutionspositionen angegeben): 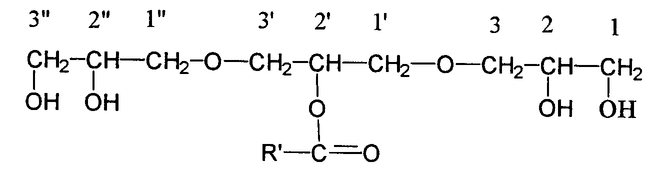
wobei R'' einen Kohlenwasserstoffrest, vorteilhaft einen verzweigten oder unverzweigten Alkyl- oder Alkenylrest Alkylrest von 5 bis 17 C-Atomen darstellt.

Die diesen Estern zugrundeliegenden Säuren sind die

| | |
|---|---|
| Hexansäure (Capronsäure) | (R' bzw. R''= -C₅H₁₁), |
| Heptansäure (Önanthsäure) | (R' bzw. R''= -C₆H₁₃), |
| Octansäure (Caprylsäure) | (R' bzw. R''= -C₇H₁₅), |
| Nonansäure (Pelargonsäure) | (R' bzw. R''= -C₈H₁₇), |
| Decansäure (Caprinsäure) | (R' bzw. R''= -C₉H₁₉), |
| Undecansäure | (R' bzw. R''= -C₁₀H₂₁), |
| 10-Undecensäure (Undecylensäure) | (R' bzw. R''= -C₁₀H₁₉), |
| Dodecansäure (Laurinsäure) | (R' bzw. R''= -C₁₁H₂₃), |
| Tridecansäure | (R' bzw. R''= -C₁₂H₂₅), |
| Tetradecansäure (Myristinsäure) | (R' bzw. R''= -C₁₃H₂₇), |
| Pentadecansäure | (R' bzw. R''= -C₁₄H29), |
| Hexadecansäure (Palmitinsäure) | (R' bzw. R''= -C₁₅H₃₁), |
| Heptadecansäure (Margarinsäure) | (R' bzw. R''= -C₁₆H₃₃), |
| Octadecansäure (Stearinsäure) | (R' bzw. R''= -C₁₇H₃₅). |

Besonders günstig werden R' und R'' gewählt aus der Gruppe der unverzweigten Alkylreste mit ungeraden C-Zahlen, insbesondere mit 9, 11 und 13 C-Atomen.

Im allgemeinen sind die Monocarbonsäure-monoester des Diglycerins denen des Triglycerins bevorzugt.

Erfindungsgemäß ganz besonders günstig sind

| | |
|---|---|
| Diglycerinmonocaprinat (DMC) | R' = 9 |
| Triglycerinmonolaurat (TML) | R'' = 11 |
| Diglycerinmonolaurat (DML) | R' = 11 |
| Triglycerinmonomyristat (TMM) | R'' = 13. |

Als bevorzugter erfindungsgemäßer Monocarbonsäuremonoester des Diglycerins hat sich das Diglycerinmonocaprinat (DMC) erwiesen.

Die erfindungsgemäßen Monocarbonsäuremonoester des Diglycerins liegen bevorzugt in 1-Stellung, die erfindungsgemäßen Monofettsäureester des Triglycerins bevorzugt in 2'-Stellung verestert vor.

Nach einer vorteilhaften Ausführungsform der vorliegenden Erfindung wird ein zusätzlicher Anteil an in anderen Stellen verestertem Di- oder Triglycerin, ebenso wie gegebenenfalls ein Anteil an den verschiedenen Diestern des Di- bzw. Triglycerins verwendet.

Insbesondere vorteilhaft sind solche Monocarbonsäureester, welche nach einem Verfahren erhältlich sind, wie es in der DE-OS 38 18 293 beschrieben wird.

Die Diglycerinester, welche sich durch zwei, und die Triglycerinester, welche sich durch drei Asymmetriezentren auszeichnen, sind in all ihren Konfigurationen erfindungsgemäß wirksam. Die Diglycerinester besitzen vier, die Triglycerinester acht Stereoisomere.

Bei den Diglycerinestern sind die 2- und die 2'-Position Asymmetriezentren. Erfindungsgemäß aktiv und gleichermaßen von Vorteil sind die 2S2'S-, die 2R2'S-, die 2S2'R- und die 2R2'R-Konfiguration.

Bei den Triglycerinestern sind die 2-, die 2' und die 2''-Position Asymmetriezentren. Erfindungsgemäß aktiv und gleichermaßen von Vorteil sind die 2S2'S2''S-, die 2R2'S2''S-, die 2S2'R2''S-, die 2R2'R2''S-, die 2S2'S2''R, die 2R2'S2''R-, die 2S2'R2''R- und die 2R2'R2''R-Konfiguration.

Es hat sich als günstig herausgestellt, racemische Gemische der Stereoisomeren zu verwenden.

Die erfindungsgemäßen Zubereitungen sind besonders vorteilhaft dadurch gekennzeichnet, daß der oder die Monocarbonsäureester des Di- und/oder Triglycerins in Konzentrationen von 0,01 - 10,00 Gew.-%, bevorzugt 0,05 - 5,00 Gew.-%, besonders bevorzugt 0,1 - 3,00 Gew.-%, jeweils bezogen auf das Gesamtgewicht der Zusammensetzung, vorliegt oder vorliegen.

Retinol ist durch folgende Struktur gekennzeichnet: 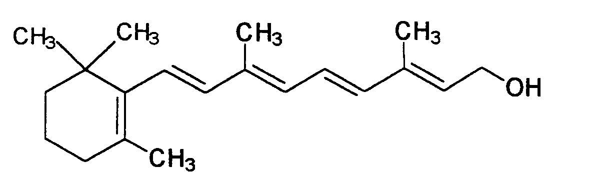
Retinol (auch: Axerophthol; [3,7-Dimethyl-9-(2,6,6-trimethyl-1-cyclohexenyl)-2,4,6,8-nonatetraen-1-ol) ist synonym mit Vitamin A₁, wird auch analog den Derivaten Retin-1-carbonsäure (Vitamin-A-Säure, Retinoësäure, Tretinoin) und deren Estern bzw. dem Retin-1-al (Vitamin-A-aldehyd) gelegentlich Vitamin-A-Alkohol genannt.

Retinol ist ein bekanntes Aknetherapeutikum.

Erfindungsgemäß gleichermaßen vorteilhaft können auch Retinolester, entweder allein, oder untereinander oder in Kombination mit unverestertem Retinol in den erfindungsgemäßen Wirkstoffkombinationen eingesetzt werden. Die Erfindungsgemäßen Retinolester haben vorzugsweise die Struktur 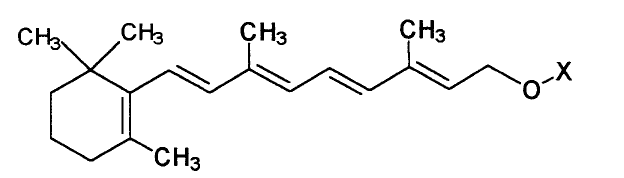
wobei X bevorzugt einen verzweigten oder unverzweigten Alkyl- oder Alkenylrest von 1 bis 25 C-Atomen darstellt. Vorzugsweise wird als Retinolester Retinolpalmitat (= Retinylpalmitat) gewählt.

Erfindungsgemäß können als günstige, aber dennoch fakultativ zu verwendende Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), sowie Koniferylbenzoat des Benzoëharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung:

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Es ist dem Fachmanne natürlich bekannt, daß anspruchsvolle kosmetische Zusammensetzungen zumeist nicht ohne die üblichen Hilfs- und Zusatzstoffe denkbar sind. Darunter zählen beispielsweise Konsistenzgeber, Füllstoffe, Parfum, Farbstoffe, Emulgatoren, zusätzliche Wirkstoffe wie Vitamine oder Proteine, Lichtschutzmittel, Stabilisatoren, Antioxidantien, Insektenrepellentien, Alkohol, Wasser, Salze, antimikrobiell, proteolytisch oder keratolytisch wirksame Substanzen usw.

Es ist besonders vorteilhaft, den erfindungsgemäßen Zubereitungen Puffersubstanzen zuzufügen. Insbesondere vorteilhaft ist, wenn die Zubereitungen auf pH-Werte von 5,5 oder kleiner abgepuffert werden.

Entsprechend können die erfindungsgemäßen Zusammensetzungen, je nach ihrem Aufbau, beispielsweise verwendet werden als Hautschutzcrème, Reinigungsmilch, Sonnenschutzlotion, Nährcrème, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Erfindungsgemäß ist auch die Verwendung der Wirkstoffkombinationen in hautpflegenden kosmetischen und/oder dermatologischen Zubereitungen.

Zur Anwendung werden die erfindungsgemäßen kosmetischen und dermatologischen Zubereitungen in der für Kosmetika üblichen Weise auf die Haut in ausreichender Menge aufgebracht.

Erfindungsgemäße kosmetische und dermatologische Zubereitungen können in verschiedenen Formen vorliegen, wie sie z.B. üblicherweise für diesen Typ von Zubereitungen eingesetzt werden. So können sie z.B. eine Lösung, eine Emulsion vom Typ Wasser-in-Öl (W/O) oder vom Typ Öl-in-Wasser (O/W), oder eine multiple Emulsionen, beispielsweise vom Typ Wasser-in-Öl-in-Wasser (W/O/W), ein Gel, einen festen Stift oder auch ein Aerosol darstellen.

Es ist auch möglich und vorteilhaft im Sinne der vorliegenden Erfindung, die erfindungsgemäßen Wirkstoffkombinationen in wäßrige Systeme bzw. Tensidzubereitungen zur Reinigung der Haut einzufügen.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die färbende Wirkung haben, Verdikkungsmittel, oberflächenaktive Substanzen, Emulgatoren, weichmachende, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Sofern die kosmetische oder dermatologische Zubereitung eine Lösung oder Lotion darstellt, können als Lösemittel verwendet werden:
- Wasser oder wäßrige Lösungen
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Erfindungsgemäße Emulsionen, z.B. in Form einer Hautschutzcreme, einer Hautlotion, einer kosmetischen Milch, beispielsweise in Form einer Sonnenschutzcreme oder einer Sonnenschutzmilch, sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Erfindungsgemäße Zubereitungen können auch günstig als Gele vorliegen, die neben den erfindungsgemäßen Wirkstoffkombinationen und dafür üblicherweise verwendeten Lösemitteln, üblicherweise Alkohole niedriger C-Zahl, z.B. Ethanol, Isopropanol, 1,2-Propandiol, Glycerin und Wasser bzw. ein vorstehend genanntes Öl, dann noch organische Verdickungsmittel enthalten, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z.B. Siliciumdioxid und/oder Aluminiumsilikate wie beispielsweise Bentonite, oder ein Gemisch aus Polyethylenglykol und Polyethylenglykolstearat oder - distearat. Das oder die Verdickungsmittel sind im Gel z.B. in einer Menge zwischen 0,1 und 30 Gew.-%, bevorzugt zwischen 0,5 und 15 Gew.-%, enthalten.

Erfindungsgemäße feste Stifte enthalten z.B. natürliche oder synthetische Wachse, Fettalkohole oder Fettsäureester.

Als Treibmittel für erfindungsgemäße, aus Aerosolbehältem versprühbare kosmetische und/oder dermatologische Zubereitungen sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die vorliegende Erfindung geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Die erfindungsgemäßen kosmetischen oder dermatologischen Zubereitungen enthalten bevorzugt
- 0,001 - 0,20 Gew.-%: Retinol und oder dessen Ester
- 0,001 - 5,00 Gew.-%: einer oder mehreren α-Hydroxycarbonsäuren und/oder α-Ketocarbonsäuren, insbesondere Citronensäure, und
- 0,001 - 10,00 Gew.-%: eines oder mehrerer Stoffe aus der Gruppe der Monoglycerinmonocarbonsäure-monoester, der Diglycerin-monocarbonsäuremonoester sowie der Triglycerin-monocarbonsäure-monoester
jeweils bezogen auf das Gesamtgewicht der Zubereitungen.

Bevorzugt können sie außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen, um kosmetische und/oder dermatologische Zubereitungen zur Verfügung zu stellen, die die Haut vor dem gesamten Bereich der ultravioletten Strahlung schützen.

Enthalten die erfindungsgemäßen Emulsionen UVB-Filtersubstanzen, können diese öllöslich oder wasserlöslich sein. Erfindungsgemäß vorteilhafte öllösliche UVB-Filter sind z.B.:
- 3-Benzylidencampher-Derivate, z.B. 3-(4-Methylbenzyliden)campher, 3-Benzylidencampher;
- 4-Aminobenzoesäure-Derivate, z.B. 4-(Dimethylamino)-benzoesäure(2-ethylhexyl)ester, 4-(Dimethylamino)benzoësäureamylester;
- Ester der Zimtsäure, z.B. 4-Methoxyzimtsäure(2-ethylhexyl)ester, 4-Methoxyzimtsäureisopentylester;
- Ester der Salicylsäure, z.B. Salicylsäure(2-ethylhexyl)ester, Salicylsäure(4-isopropylbenzyl)ester, Salicylsäurehomomenthylester,
- Derivate des Benzophenons, z.B. 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, z.B. 4-Methoxybenzalmalonsäuredi(2-ethylhexyl)ester, -2,4,6-Trianilino-(p-carbo-2'-ethyl-1'-hexyloxy) -1,3,5-triazin.

Vorteilhafte wasserlösliche UVB-Filter sind z.B.:
- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, wie z.B. 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze.

Enthalten die erfindungsgemäßen Emulsionen UVA-Filtersubstanzen, können diese erfindungsgemäß vorteilhaft gewählt werden aus der Gruppe der Derivate des Dibenzoylmethans, z.B. 1-(4'-tert.Butylphenyl)-3-(4'-methoxyphenyl)propan-1,3-dion und 1-Phenyl-3-(4'-isopropylphenyl)propan-1,3-dion.

Die erfindungsgemäßen Zubereitungen können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen bzw. Abwandlungen davon. Besonders bevorzugt handelt es sich um Pigmente auf der Basis von Titandioxid.

Bei erfindungsgemäßen kosmetischen und dermatologischen Zubereitungen für die Verwendung an behaarter Haut handelt es sich beispielsweise um Shampoonierungsmittel, Zubereitungen, die beim Spülen der Haare vor oder nach der Shampoonierung, vor oder nach der Dauerwellbehandlung, vor oder nach der Färbung oder Entfärbung der Haare angewendet werden, um Zubereitungen zum Fönen oder Einlegen der Haare, Zubereitungen zum Färben oder Entfärben, um eine Frisier- und Behandlungslotion, einen Haarlack oder um Dauerwellmittel.

Die kosmetischen und/oder dermatologischen Zubereitungen enthalten gegebenenfalls zusätzliche Wirkstoffe, Hilfs- und/oder Zusatzstoffe, wie sie üblicherweise für diesen Typ von Zubereitungen zur Haarpflege und Haarbehandlung verwendet werden. Als Hilfsstoffe dienen Konservierungsmittel, oberflächenaktive Substanzen, Substanzen zum Verhindern des Schäumens, Emulgatoren, Verdickungsmittel, Fette, Öle, Wachse, organische Lösungsmittel, Bakterizide, Parfüme, Farbstoffe oder Pigmente, deren Aufgabe es ist, die Haare oder die kosmetische oder dermatologische Zubereitung selbst zu färben, Elektrolyte sowie zusätzliche Substanzen zum Rückfetten der Haare bzw. der Kopfhaut.

Erfindungsgemäße wäßrige kosmetische Reinigungsmittel mit einem Gehalt an den erfindungsgemäßen Wirkstoffkombinationen bzw. für die wäßrige Reinigung bestimmte wasserarme oder wasserfreie Reinigungsmittelkonzentrate können anionische, nichtionische und/oder amphotere Tenside enthalten, beispielsweise
- herkömmliche Seifen, z.B. Fettsäuresalze des Natriums
- Alkylsulfate, Alkylethersulfate, Alkan- und Alkylbenzolsulfonate
- Sulfoacetate
- Sulfobetaine
- Sarcosinate
- Amidosulfobetaine
- Sulfosuccinate
- Sulfobernsteinsäurehalbester
- Alkylethercarboxylate
- Eiweiß-Fettsäure-Kondensate
- Alkylbetaine und Amidobetaine
- Fettsäurealkanolamide
- Polyglycolether-Derivate
Kosmetische Zubereitungen, die kosmetische Reinigungszubereitungen für die Haut darstellen, können in flüssiger oder fester Form vorliegen. Sie enthalten vorzugsweise mindestens eine anionische, nicht-ionische oder amphotere oberflächenaktive Substanz oder Gemische daraus, mindestens einen erfindungsgemäßes Elektrolyten und Hilfsmittel, wie sie üblicherweise dafür verwendet werden. Die oberflächenaktive Substanz kann in einer Konzentration zwischen 1 und 94 Gew.-% in den Reinigungszubereitungen vorliegen, bezogen auf das Gesamtgewicht der Zubereitungen.

Kosmetische Zubereitungen, die ein Shampoonierungsmittel darstellen, enthalten neben einem wirksamen Gehalt an den erfindungsgemäßen Wirkstoffkombinationen vorzugsweise mindestens eine anionische, nicht-ionische oder amphotere oberflächenaktive Substanz oder Gemische daraus, gegebenenfalls einen erfindungsgemäßes Elektrolyten und Hilfsmittel, wie sie üblicherweise dafür verwendet werden. Die oberflächenaktive Substanz kann in einer Konzentration zwischen 1 Gew.-% und 94 Gew.-% in dem Shampoonierungsmittel vorliegen.

Liegen die kosmetischen oder dermatologischen Zubereitungen in Form einer Lotion vor, die ausgespült und z.B. vor oder nach der Entfärbung, vor oder nach der Shampoonierung, zwischen zwei Shampoonierungsschritten, vor oder nach der Dauerwellbehandlung angewendet wir, so handelt es sich dabei z.B. um wäßrige oder wäßrig-alkoholische Lösungen, die gegebenenfalls oberflächenaktive Substanzen enthalten, bevorzugt nicht-ionische oder kationische oberflächenaktive Substanzen, deren Konzentration zwischen 0,1 und 10 Gew.-%, vorzugsweise zwischen 0,2 und 5 Gew.-%, liegen kann. Diese kosmetischen und/oder dermatologischen Zubereitungen können auch Aerosole mit den üblicherweise dafür verwendeten Hilfsmitteln darstellen.

Eine kosmetische Zubereitung in Form einer Lotion, die nicht ausgespült wird, insbesondere eine Lotion zum Einlegen der Haare, eine Lotion, die beim Fönen der Haare verwendet wird, eine Frisier- und Behandlungslotion, stellt im allgemeinen eine wäßrige, alkoholische oder wäßrig-alkoholische Lösung dar und enthält mindestens ein kationisches, anionisches, nicht-ionisches oder amphoteres Polymer oder auch Gemische derselben, sowie die erfindungsgemäßen Wirkstoffkombinationen. Die Menge der verwendeten Polymeren liegt z.B. zwischen 0,1 und 10 Gew.-%, bevorzugt zwischen 0,1 und 3 Gew.-%.

Erfindungsgemäße kosmetische Zubereitungen zur Behandlung und Pflege der behaarten Haut, die erfindungsgemäße Wirkstoffkombinationen enthalten, können als Emulsionen vorliegen, die vom nicht-ionischen oder anionischen Typ sind. Nicht-ionische Emulsionen enthalten neben Wasser Öle oder Fettalkohole, die beispielsweise auch polyethoxyliert oder polypropoxyliert sein können, oder auch Gemische aus den beiden organischen Komponenten. Diese Emulsionen enthalten gegebenenfalls kationische oberflächenaktive Substanzen.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Sofern die kosmetische und/oder dermatologische Zubereitung eine Lösung oder Lotion darstellt, können als Lösungsmittel verwendet werden:
- Wasser oder wäßrige Lösungen
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder - monobutylether, Diethylenglykolmonomethyl- oder -monoethylether und analoge Produkte.

Insbesondere werden Gemische der vorstehend genannten Lösemittel verwendet. Bei alkoholischen Lösemitteln kann Wasser ein weiterer Bestandteil sein.

Erfindungsgemäße Emulsionen zur Nagelpflege enthalten z.B. die genannten Fette, Öle, Wachse und andere Fettkörper, sowie Wasser und einen Emulgator, wie er üblicherweise für einen solchen Typ der Formulierung verwendet wird.

Erfindungsgemäße Gele zur Pflege und/oder Wiederherstellung der Nägel enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdikkungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Ansonsten gelten für diese Gruppe an kosmetischen und/oder dermatologischen Zubereitungen die üblichen Anforderungen, die der Fachmann an solche Zubereitungen und deren Inhaltsstoffe stellt.

Die folgenden Beispiele wollen die vorliegende Erfindung erläutern, ohne daß eine Beschränkung auf den Gehalt der Beispiele beabsichtigt ist. Die Mengenangaben bedeuten stets, sofern nichts Anderes angegeben ist, Gewichts-%, bezogen auf das Gesamtgewicht der jeweiligen Zubereitungen.

### Beispiel 1

### Gelcrème ohne UV-Schutz

| Phase | Rohstoff | Gew.-% |
|---|---|---|
| 2 | PPG-14 Butylether | 4,00 |
| 1 | Glycerin | 3,00 |
| 1 | Salicylsäure | 2,00 |
| 2 | Stearylalkohol | 2,00 |
| 2 | Mineralöl, DAB 9 | 1,00 |
| 2 | PPG-1 Trideceth-6 | 1,00 |
| 2 | Cyclomethicon | 3,50 |
| 1 | GMC | 0,50 |
| 1 | DMC | 1,00 |
| 2 | Steareth-21 | 1,50 |
| 2 | Steareth-2 | 1,50 |
| 1 | NaOH | 0,80 |
| 3 | Parfum | q.s. |
| 1 | Wasser | ad 100,00 |

Phase 1 und 2 werden in getrennten Behältern auf 75 - 80° C erhitzt, wobei die Feststoffe gelöst sein sollen. Nach Vereinigung beider Phasen wird bei 65° C homogenisiert und die Emulsion auf 35° C abgekühlt. Sodann wird Phase 3 zugegeben. Bei 30° C wird nochmals homogenisiert.

### Beispiel 2

### Gelcrème mit UV-Schutz

| Phase | Rohstoff | Gew.-% |
|---|---|---|
| 2 | PPG-14 Butylether | 4,00 |
| 1 | Glycerin | 3,00 |
| 2 | Stearylalkohol | 2,00 |
| 2 | Octylmethoxycinnamat | 2,00 |
| 2 | Butylmethoxydibenzoylmethan | 0,20 |
| 2 | Mineralöl, DAB 9 | 1,00 |
| 2 | PPG-1 Trideceth-6 | 1,00 |
| 2 | Cyclomethicon | 3,50 |
| 1 | Salicylsäure | 2,00 |
| 1 | GMC | 0,50 |
| 1 | DMC | 1,00 |
| 2 | Steareth-21 | 1,50 |
| 2 | Steareth-2 | 1,50 |
| 1 | NaOH | 0,80 |
| 3 | Parfum | q.s. |
| 1 | Wasser | ad 100,00 |

Phase 1 und 2 werden in getrennten Behältern auf 75 - 80° C erhitzt, wobei die Feststoffe gelöst sein sollen. Nach Vereinigung beider Phasen wird bei 65° C homogenisiert und die Emulsion auf 35° C abgekühlt. Sodann wird Phase 3 zugegeben. Bei 30° C wird nochmals homogenisiert.

### Beispiel 3

### Gesichtspflegegel, klar

| Phase | Rohstoff | Gew.-% |
|---|---|---|
| 1 | PEG-150 | 1,00 |
| 1 | Butylenglycol | 8,00 |
| 1 | Carbomer | 1,00 |
| 2 | NaOH | 0,70 |
| 1 | Milchsäure | 2,00 |
| 1 | GMC | 0,50 |
| 1 | DMC | 1,00 |
| 1 | Hamamelisextrakt | 2,00 |
| 1 | Konservierungsmittel | q.s. |
| 3 | Parfum | q.s. |
| 1 | Wasser | ad 100,00 |

Phase 1 wird bei 75 - 80° C klar gelöst und langsam gerührt, unter Zugabe von Phase 2. Unter ständigem Rühren wird das gebildete Gel auf 35° C abgekühlt und Phase 3 hinzugegeben.

### Beispiel 4

### Abdeckcrème

| Phase | Rohstoff | Gew.-% |
|---|---|---|
| 2 | PEG-12 | 4,00 |
| 1 | Butylenglycol | 5,00 |
| 2 | Steareth-2 | 2,00 |
| 3 | TiO₂ | 19,00 |
| 2 | Myristylalkohol | 2,50 |
| 1 | Acrylat/C₁₀₋₃₀-Alkylacrylat Copolymer | 0,50 |
| 1 | Carbomer | 0,30 |
| 1 | GMC | 0,50 |
| 1 | DMC | 1,00 |
| 1 | Konservierungsmittel | q.s. |
| 3 | Parfum | q.s. |
| 1 | Wasser | ad 100,00 |

Phase 1 und 2 werden in getrennten Behältern auf 75 - 80° C erhitzt, wobei die Feststoffe gelöst sein sollen. Nach Vereinigung beider Phasen wird bei 65° C homogenisiert und die Emulsion auf 35° C abgekühlt. Sodann wird Phase 3 zugegeben. Bei 30° C wird nochmals homogenisiert.

### Beispiel 5

### O/W-Lotion ohne UV-Schutz

| Phase | Rohstoff | Gew.-% |
|---|---|---|
| 2 | Caprylic/Capric Triglycerid | 2,00 |
| 2 | Mineralöl DAB 9 | 1,00 |
| 1 | Sorbitol | 3,00 |
| 2 | Cetearylalkohol | 2,00 |
| 2 | Ceteareth-20 | 1,50 |
| 2 | Ceteareth-12 | 1,50 |
| 1 | Salicylsäure | 2,00 |
| 1 | GML | 0,50 |
| 1 | DML | 1,00 |
| 1 | Carbomer | 0,30 |
| 3 | NaOH | 0,80 |
| 4 | Konservierungsmittel | q.s. |
| 4 | Parfum | q.s. |
| 1 | Wasser | ad 100,00 |

Phase 1 und 2 werden in getrennten Behältern auf 75 - 80° C erhitzt, wobei die Feststoffe gelöst sein sollen. Nach Vereinigung beider Phasen wird mit Phase 3 unter ständigem Rühren neutralisiert. Bei 65° C Phasentemperatur wird homogenisiert und die Emulsion auf 35° C abgekühlt. Sodann wird Phase 4 zugegeben. Bei 30° C wird nochmals homogenisiert.

### Beispiel 6

### O/W-Lotion mit UV-Schutz

| Phase | Rohstoff | Gew.-% |
|---|---|---|
| 2 | Caprylic/Capric Triglycerid | 2,00 |
| 2 | Mineralöl DAB 9 | 1,00 |
| 2 | Octylmethoxycinnamat | 2,00 |
| 2 | Butylmethoxydibenzoylmethan | |
| 1 | Sorbitol | 3,00 |
| 2 | Cetearylalkohol | 2,00 |
| 2 | Ceteareth-20 | 1,50 |
| 2 | Ceteareth-12 | 1,50 |
| 1 | Salicylsäure | 2,00 |
| 1 | GML | 0,50 |
| 1 | DML | 1,00 |
| 1 | Carbomer | 0,30 |
| 3 | NaOH | 0,80 |
| 4 | Konservierungsmittel | q.s. |
| 4 | Parfum | q.s. |
| 1 | Wasser | ad 100,00 |

Phase 1 und 2 werden in getrennten Behältern auf 75 - 80° C erhitzt, wobei die Feststoffe gelöst sein sollen. Nach Vereinigung beider Phasen wird mit Phase 3 unter ständigem Rühren neutralisiert. Bei 65° C Phasentemperatur wird homogenisiert und die Emulsion auf 35° C abgekühlt. Sodann wird Phase 4 zugegeben. Bei 30° C wird nochmals homogenisiert.

### Beispiel 7

### Getönte Gelcrème ohne UV-Schutz

| Phase | Rohstoff | Gew.-% |
|---|---|---|
| 2 | Cyclomethicon | 5,00 |
| 1 | Steareth-20 | 2,00 |
| 1 | Butylenglycol | 5,00 |
| 2 | Cetearylalkohol | 2,00 |
| 1 | Xanthangummi | 0,40 |
| 2 | Carbomer | 0,80 |
| 1 | Salicylsäure | 2,00 |
| 1 | GML | 0,50 |
| 2 | DML | 1,00 |
| 3 | NaOH | 0,80 |
| 4 | TiO₂ | 0,35 |
| 4 | Farbpigmente | q.s. |
| 5 | Parfum | q.s. |
| 1 | Wasser | ad 100,00 |

Phase 1 und 2 werden in getrennten Behältern auf 75 - 80° C erhitzt, wobei die Feststoffe gelöst sein sollen. Nach Vereinigung beider Phasen wird mit Phase 3 unter ständigem Rühren neutralisiert. Anschließend wird Phase 4 eingestreut. Bei 65° C Phasentemperatur wird homogenisiert und die Emulsion auf 35° C abgekühlt. Sodann wird Phase 5 zugegeben. Bei 30° C wird nochmals homogenisiert.

### Beispiel 8

### Getönte Gelcrème mit UV-Schutz

| Phase | Rohstoff | Gew.-% |
|---|---|---|
| 2 | Cyclomethicon | 5,00 |
| 1 | Steareth-20 | 2,00 |
| 1 | Butylenglycol | 5,00 |
| 2 | Cetearylalkohol | 2,00 |
| 1 | Xanthangummi | 0,40 |
| 2 | Carbomer | 0,80 |
| 2 | Octylmethoxycinnamat | 2,00 |
| 2 | Butylmethoxydibenzoylmethan | 0,20 |
| 1 | Salicylsäure | 2,00 |
| 1 | GML | 0,50 |
| 2 | DML | 1,00 |
| 3 | NaOH | 0,80 |
| 4 | TiO₂ | 0,35 |
| 4 | Farbpigmente | q.s. |
| 5 | Parfum | q.s. |
| 1 | Wasser | ad 100,00 |

Phase 1 und 2 werden in getrennten Behältern auf 75 - 80° C erhitzt, wobei die Feststoffe gelöst sein sollen. Nach Vereinigung beider Phasen wird mit Phase 3 unter ständigem Rühren neutralisiert. Anschließend wird Phase 4 eingestreut. Bei 65° C Phasentemperatur wird homogenisiert und die Emulsion auf 35° C abgekühlt. Sodann wird Phase 5 zugegeben. Bei 30° C wird nochmals homogenisiert.

### Beispiel 9

### Akne-Gesichtswasser, alkoholfrei

| Phase | Rohstoff | Gew.-% |
|---|---|---|
| 1 | Lecithin | 1,00 |
| 1 | Wasser | 15,00 |
| 2 | GMC | 0,50 |
| 2 | DMC | 1,0 |
| 2 | Butylenglycol | 4,00 |
| 2 | Natriumlaurethsulfat | 4,00 |
| 2 | Milchsäure | 2,00 |
| 3 | Parfum | q.s. |
| 3 | Wasser | ad 100,00 |

Phase 2 wird vermischt und auf 60° C erwärmt, sodann auf 50° C abgekühlt. Dann wird Phase 1 langsam und unter Rühren hinzugegeben. Das Phasengeimsch wird auf 35° C abgekühlt, dann wird Phase 3 untergerührt.

### Beispiel 10

### Akne-Gesichtswasser, alkoholfrei

| Phase | Rohstoff | Gew.-% |
|---|---|---|
| 1 | Lecithin | 1,00 |
| 1 | Wasser | 15,00 |
| 2 | GML | 0,50 |
| 2 | Butylenglycol | 4,00 |
| 2 | Natriumlaurethsulfat | 4,00 |
| 2 | Milchsäure | 2,00 |
| 3 | Parfum | q.s. |
| 3 | Wasser | ad 100,00 |

Phase 2 wird vermischt und auf 60° C erwärmt, sodann auf 50° C abgekühlt. Dann wird Phase 1 langsam und unter Rühren hinzugegeben. Das Phasengeimsch wird auf 35° C abgekühlt, dann wird Phase 3 untergerührt.

### Beispiel 11

### Akne-Gesichtswasser, alkoholhaltig

| Phase | Rohstoff | Gew.-% |
|---|---|---|
| 1 | GMC | 0,50 |
| 1 | DMC | 1,00 |
| 1 | Butylenglycol | 4,00 |
| 1 | Natriumlaurethsulfat | 4,00 |
| 1 | Wasser | ad 100,00 |
| 2 | Ethanol | 20,00 |
| 2 | Salicylsäure | 2,00 |
| 2 | Retinylpalmitat | 0,40 |
| 2 | Parfum | q.s. |

Salicylsäure und Retinylpalmitat werden vollständig im Alkohol gelöst, sodann wird gewünschtenfalls das Parfum zugegeben (Phase 2). Die Bestandteile der Phase 1 wird auf 60° C erwärmt und verrührt. Nach Abkühlen auf 35° C wird unter Rühren Phase 2 hinzugegeben.

### Beispiel 12

### Akne-Gesichtswasser, alkoholhaltig

| Phase | Rohstoff | Gew.-% |
|---|---|---|
| 1 | GMC | 0,50 |
| 1 | DMC | 1,00 |
| 1 | Butylenglycol | 10,00 |
| 1 | Natriumlaurethsulfat | 2,00 |
| 1 | Wasser | ad 100,00 |
| 2 | Ethanol | 10,00 |
| 2 | Milchsäure | 2,00 |
| 2 | Retinylpalmitat | 0,40 |
| 3 | Lecithin | 1,00 |
| 3 | Wasser | 15,00 |
| 2 | Parfum | q.s. |

Milchsäure und Retinylpalmitat werden vollständig im Alkohol gelöst, sodann wird gewünschtenfalls das Parfum zugegeben (Phase 2). Die Bestandteile der Phase 1 wird auf 60° C erwärmt und verrührt. Nach Abkühlen auf 50° C wird Phase 3 hinzugegeben. Nach weiterem Abkühlen auf 30° C wird unter Rühren Phase 2 hinzugegeben.

### Beispiel 13

### Akne-Gesichtswasser, alkoholhaltig

| Phase | Rohstoff | Gew.-% |
|---|---|---|
| 1 | DML | 1,50 |
| 1 | Butylenglycol | 10,00 |
| 1 | Natriumlaurethsulfat | 2,00 |
| 1 | Wasser | ad 100,00 |
| 2 | Ethanol | 10,00 |
| 2 | Milchsäure | 2,00 |
| 2 | Retinylpalmitat | 0,40 |
| 3 | Lecithin | 1,00 |
| 3 | Wasser | 15,00 |
| 2 | Parfum | q.s. |

Milchsäure und Retinylpalmitat werden vollständig im Alkohol gelöst, sodann wird gewünschtenfalls das Parfum zugegeben (Phase 2). Die Bestandteile der Phase 1 wird auf 60° C erwärmt und verrührt. Nach Abkühlen auf 50° C wird Phase 3 hinzugegeben. Nach weiterem Abkühlen auf 30° C wird unter Rühren Phase 2 hinzugegeben.

## Patentansprüche

1. Kosmetische und/oder dermatologische Wirkstoffkombinationen, bestehend aus
a) einer wirksamen Menge an Salicylsäure und/oder einer wirksamen Menge an einer oder mehreren α-Hydroxycarbonsäuren und/oder einer wirksamen Menge an einer oder mehreren α-Ketocarbonsäuren
b) einem oder mehreren Stoffen, gewählt aus der Gruppe der Monoglycerin-monocarbonsäure-monoester, der Diglycerin-monocarbonsäure-monoester sowie der Triglycerin-monocarbonsäure-monoester sowie
c) gegebenenfalls einer wirksamen Menge an Retinol und/oder Derivaten des Retinols.

2. Wirkstoffkombinationen nach Anspruch 1, dadurch gekennzeichnet, daß die α-Hydroxycarbonsäure oder die α-Hydroxycarbonsäuren gewählt werden aus der Gruppe der Substanzen der allgemeinen Formel 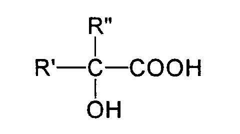 und daß die α-Ketocarbonsäure oder die α-Ketocarbonsäuren gewählt werden aus der Gruppe der Substanzen der allgemeinen Formel 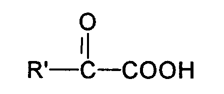 wobei jeweils R' und R'' unabhängig voneinander gewählt werden aus der Gruppe
(a1) H-,
(a2) verzweigtes oder unverzweigtes C₁₋₂₅-Alkyl-,
(a3) mit einer oder mehreren Carboxylgruppen und/oder Hydroxygruppen und/oder Aldehydgruppen und/oder Oxogruppen (Ketogruppen) substituiertes verzweigtes oder unverzweigtes C₁₋₂₅-Alkyl-
(a4) Phenyl-,
(a5) mit einer oder mehreren Carboxylgruppen und/oder Hydroxygruppen und/oder verzweigten und/oder unverzweigten C₁₋₂₅-Alkylgruppen substituiertes Phenyl-, oder wobei das α-Kohlenstoffatom der α-Hydroxycarbonsäure mit R' und R'' zusammen eine
(a6) unsubstituierte Cycloalkylgruppe mit 3 bis 7 Ringatomen oder eine
(a7) mit einer oder mehreren Carboxylgruppen und/oder Hydroxygruppen und/oder Oxogruppen (Ketogruppen) und/oder verzweigten und/oder unverzweigten C₁₋₂₅-Alkylgruppen substituierte Cycloalkylgruppe mit 3 bis 7 Ringatomen
ausbildet und
wobei die α-Hydroxycarbonsäure und/oder die α-Hydroxycarbonsäuren und/oder die α-Ketocarbonsäure und/oder die α-Ketocarbonsäuren gegebenenfalls in Form ihrer physiologisch verträglichen Salze und/oder Ethylester und/oder Methylester vorliegen können.

3. Wirkstoffkombinationen nach Anspruch 1, dadurch gekennzeichnet, daß die α-Hydroxysäuren gewählt werden aus der Gruppe α-Hydroxyfettsäuren, α-Hydroxyzuckersäuren, aliphatische α-Hydroxyfruchtsäuren, unsubstituierte aromatische α-Hydroxycarbonsäuren, substituierte aromatische α-Hydroxycarbonsäuren.

4. Wirkstoffkombinationen nach Anspruch 1, dadurch gekennzeichnet, daß die α-Hydroxysäuren gewählt werden aus der Gruppe
- α-Hydroxycarbonsäuren, gemäß der Formel 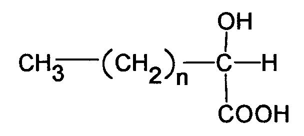 und/oder
- α-Hydroxy-isocarbonsäuren, gemäß der Formel 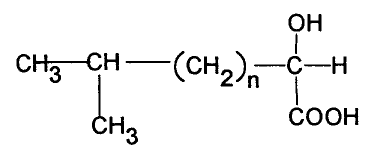 und/oder
- α-Hydroxy-anteisocarbonsäuren, gemäß der Formel 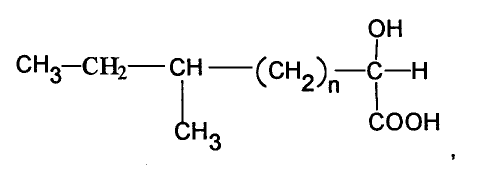 wobei n jeweils eine Zahl von 7 bis 31 darstellt, sowie
- Aldonsäuren, Aldarsäuren, Uronsäuren, Glycerinsäure, Äpfelsäure, Milchsäure, Essigsäure, Citronensäure.

5. Wirkstoffkombinationen nach Anspruch 1, dadurch gekennzeichnet, daß die Substanz oder die Substanzen der Gruppe der Monoglycerin-monocarbonsäure-monoester, der Diglycerin-monocarbonsäure-monoester und der Triglycerin-monocarbonsäure-monoester gewählt wird aus der Gruppe Glycerinmonocaprylat, Glycerinmonocaprinat, Diglycerinmonocaprinat, Triglycerinmonolaurat, Diglycerinmonolaurat, Triglycerinmonomyristat.

6. Wirkstoffkombinationen nach Anspruch 1, dadurch gekennzeichnet, daß die Retinolderivate gewählt werden aus der Gruppe der Substanzen folgender Struktur: 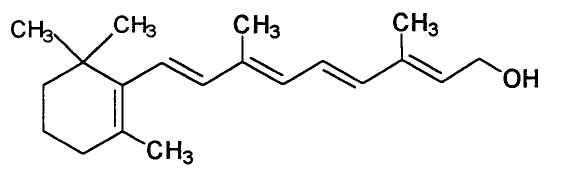 wobei X bevorzugt einen verzweigten oder unverzweigten Alkyl- oder Alkenylrest von 1 bis 25 C-Atomen darstellt.

7. Wirkstoffkombinationen nach Anspruch 1, dadurch gekennzeichnet, daß als Retinolester Retinolpalmitat (= Retinylpalmitat) gewählt wird.

8. Verwendung von Wirkstoffkombinationen nach Anspruch 1 zur Bekämpfung unreiner Haut, leichter Formen der Akne sowie Propionibakterium acnes.
